# EUROPEAN PATENT SPECIFICATION

(11) **EP 0 584 256 B1**
(45) Date of publication and mention of the grant of the patent: **10.11.1999**
(21) Application number: 92912903.9
(22) Date of filing: 22.04.1992
(51) Int. Cl.: C07K 7/08, A61K 51/00, G01N 33/68

(54) **COMPOUNDS AND PHARMACEUTICAL COMPOSITIONS FOR DETECTING AND LOCALIZING TISSUES HAVING NEUROKININE 1 RECEPTORS**
VERBINDUNGEN UND PHARMAZEUTISCHE ZUSAMMENSETZUNGEN ZUM NACHWEIS UND ZUR LOKALISIERUNG VON GEWEBEN MIT NEUROKININ-1-REZEPTOREN
COMPOSES ET COMPOSITIONS POUR LA DETECTION ET LA LOCLISATION DE TISSUS COMPRENANT DES RECEPTEURS DE NEUROKININE 1

(30) Priority: 22.04.1991 EP 91200955
(43) Date of publication of application: 02.03.1994
(73) Proprietor: MALLINCKRODT MEDICAL, INC., St. Louis, MO 63134 (US)
(72) Inventor: VISSER, Theofilus, J. Octrooibureau Zoan B.V., NL-Weesp (NL); LAMBERTS, Steven, W., J. Octrooibureau Zoan B.V., NL-Weesp (NL); KRENNING, Eric, P. Octrooibureau Zoan B.V., NL-Weesp (NL); BAKKER, Willem, H. Octrooibureau Zoan B.V., NL-Weesp (NL); VAN HAGEN, Petrus, M. Octrooibureau Zoan B.V., NL-Weesp (NL)
(74) Representative: Eyles, Christopher Thomas
(86) International application number: US9203307
(87) International publication number: WO9218536

(56) References cited:
- EP-A- 0 165 630
- EP-A- 0 176 436
- EP-A- 0 347 802
- WO-A-87/07643
- WO-A-89/07456
- US-A- 4 481 139
- EUROPEAN JOURNAL OF PHARMACOLOGY vol. 184, 1990, pages 97-108, GB, R. LEW et al.: "Binding characteristics of (125I) Bolton-Hunter (Sar9, Met(O2)11) substance P, a new selective radioligand for the NK1 receptor", see pages 106,107 (cited in the application)
- BRAIN RESEARCH, vol. 524, 1990, pages 263-270, GB, C. TOUSIGNANT et al.: "125I-BH (Sar9, Met(O2)11)-SP, a new selective ligand for the NK-1 receptor in the central nervous system" (cited in the application)
- BIOCHEMICAL PHARMACOLOGY, vol. 37, no. 1, 1988, pages 41-49, GB, S. LAVIELLE et al.: "Analysis of Tachykinin binding site interactions using constrained analogues of Takykinins", see page 41, column 2 - page 42, column 1 (cited in the application)
- PHARMACOLOGY, vol. 38, 1989, pages 1-15, CH, D. REGOLI et al.: "Receptors for substance P and related neurokinins", see pages 4-6 (cited in the application)

## Description

The invention relates to compounds and pharmaceutical compositions for detecting and localizing tissues having neurokinine 1 receptors in the body of a warm-blooded living being. The invention also relates to compounds and compositions which can be used in the therapeutic treatment of tumors having on their surface neurokinine 1 receptors in the body of said being. The invention further relates to a kit for preparing a radiopharmaceutical composition.

Binding studies performed in vitro have demonstrated that small peptides that participate in the regulation of diverse biological processes, as well as analogues thereof, have a high affinity to certain tissues. Neurokinine 1 (NK1) receptors are demonstrated in both the brain and peripheral tissues. Such tissues prefer interaction with certain small peptides such as substance P (SP) and related compounds. In these studies ¹²⁵I-Bolton Hunter derivatized analogues of SP and related compounds were used to determine the binding affinity of such small peptides to NK1 specific binding sites. A number of recent publications is devoted to these in vitro studies, wherein different tissue preparations of test animals are used, e.g. preparations of brain membranes, of synaptosomes, of duodenal membranes, of urinary bladders, of ilia, of carotid arteries and of salivary glands. Reference is made in this connection to the publications of Lavielle et al. in Biochem. Pharmacol. 37, 1988, 41-49; Regoli et al. in Pharmacology 38, 1989, 1-15; Lew et al. in Eur. J. Pharmacol. 184, 1990, 97-108; and Tousignant et al. in Brain Research 524, 1990, 263-270.

It is the object of the present invention to provide compounds and pharmaceutical compositions for detecting and localizing tissues having neurokinine 1 receptors in the body of a warm-blooded living being. Such compounds and pharmaceutical compositions would be powerful tools in diagnosing various diseases and disorders, that are related to neurokinine 1 receptors in body tissues, in vivo, such as tumors with NK1 receptors, e.g. malignant glioma, pheochromocytoma, paraganglia and SCLC (small cell lung cancer), and in visualising NK1 receptors on certain tissues, such as regenerating nervous tissue, e.g. polyneuropathy, nervous section and other degenerative processes, in the central nervous system, including the spinal cord, and on tissue which shows an immunological reaction, e.g. in case of granuloma, lymphoma and Crohn's disease. In order to be able to achieve a specific therapy for the above diseases and disorders, the detection and localization of tissues having NK1 receptors in an early stage is of the utmost importance. In addition, a good diagnostic method is also indispensable for supporting the therapy used. Various requirements have to be imposed on an agent that is used in such a diagnostic method, for example, nontoxic, no adverse influence on the host resistance and/or the therapeutic treatment, well detectable and highly selective. The required high selectivity means that the diagnostic agent, after having been introduced into the body, must accumulate more strongly in the tissue or tissues to be detected or visualized than in surrounding tissues. This selectivity, i.e. a comparatively stronger concentration of the diagnostic agent in the target tissue or tissues compared with non-target tissues, enables the user to correctly diagnose the disease or disorder. In order to be detectable from outside the body, the diagnostic agent should be labelled, preferably with a radionuclide or with a paramagnetic metal isotope. In the former case, the radioactive radiation can be detected by using a suitable detector (scanning). Modern techniques in this field use emission tomography; when gamma radiating isotopes are used, the so-called single photon emission computerized tomography (SPECT) may be applied. The use of paramagnetic diagnostic agents enables a detection by means of imaging by magnetic resonance.

According to the present invention there is provided a compound which is a labelled small peptide having a selective affinity to neurokinine 1 receptors, wherein said small peptide is derived from a compound of the general formula

R₁-(A₁)ₘ-A₂)ₙ-A₃)ₒ-Pro)ₚ-A₄)_{q}-A₅-Phe-A₆-A₇-A₈-Met (O)ₛ-R₂

wherein all of the symbols m, n, o, p and q are 1, or all but one of the symbols m, n, o, p and q are 1 and the remaining symbol is zero;
R₁ is a hydrogen atom or a C₁-C₄ alkylcarbonyl group;
R₂ is an amino group, a hydroxy group or a C₁-C₄ alkoxy group;
A₁ is Arg, Gly or 5-oxo-Pro (pGlu);
A₂ is Pro or β-Ala;
A₃ is Lys or Asp;
A₄ is Gln, Asn or 5-oxo-Pro;
A₅ is Gln, Lys, Arg, N-acylated Arg or 5-oxo-Pro; or
wherein
A₅ together with A₃ forms a cystine moiety;
A₆ is Phe or Tyr;
A₇ is Gly, Sar or Pro;
A₈ is Leu or Pro; and
S is zero, 1 or 2;
or a Tyr^{o} derivative thereof;
wherein said peptide is labelled with (a) a metal isotope selected from Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, Re-186, Re-188, As-77, Y-90, Cu-67, Er-169, Sn-121, Te-127, Pr-142, Pr-143, Au-198, Pd-109 and Dy-165, said metal isotope being attached to said peptide via a linker capable of reacting with an amino group of said peptide, and having a chelating group for chelating said metal isotope, and derived from ethylene diamine tetraacetic acid (EDTA), diethylene triamine pentaacetic acid (DPTA), ethylene glycol-O,O'-bis(2-aminoethyl)-N,N,N', N'-tetraacetic acid (EGTA), N,N'-bis(hydroxybenzyl) ethylenediamine-N,N'-diacetic acid (HBED), triethylenetetramine hexaacetic acid (TTHA), substituted EDTA or substituted DTPA, 1,4,7,10-tetra-azacyclododecane-N, N', N'', N'''-tetraacetic acid (DOTA) or 1,4,8,11-tetraazacyclotetradecane-N,N',N'',N'''-tetraacetic acid (TETA) or via a linker derived from a compound of the general formula 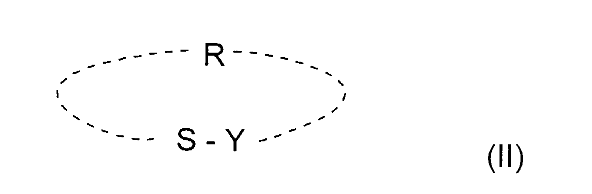 wherein R is a branched or non-branched, optionally substituted hydrocarbyl radical, which may be interrupted by one or more hetero-atoms selected from N, O and S and/or by one or more NH groups, and Y is a group which is capable of reacting with an amino group of the peptide and which is preferably selected from carbonyl, carbimidoyl, N-(C₁-C₆)-alkylcarbimidoyl, N-hydroxycarbimidoyl and N-(C₁-C₆) alkoxycarbimidoyl; or with (b) a detectable halogen radioisotope selected from I-123, I-131, Br-75 and Br-76, said halogen radioisotope being attached to said peptide directly or via a linking tyrosyl group.

Such compounds can be used in a method, which comprises (i) administering to a warm-blooded living being a composition comprising, in a quantity sufficient for external imaging, a small peptide of the above general formula labelled with (a) a detectable metal isotope selected from the group consisting of Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52 and Fe-56, said metal isotope being attached to said peptide via a suitable linker capable of reacting with an amino group, preferably a terminal amino group, of said peptide, and having a chelating group for chelating said metal isotope, or with (b) a detectable halogen radioisotope selected from I-123, I-131, Br-75 and Br-76, said halogen radioisotope being attached to said peptide directly or via a tyrosyl linking group; and thereupon (ii) subjecting said being to external imaging to determine the targeted sites in the body of said being in relation to the background activity, in order to allow detection and localization of said tissues in said body.

The above labelled peptides, having a selective affinity to neurokinine 1 receptors, have been tested in a number of suitable model experiments that are predictive for in vivo application. These experiments are described in the examples. From the results it will be evident, that the tested labelled peptides have properties which make them suitable as diagnostic agents. As will become apparent from the Examples, the labelled peptide remains sufficiently long intact after administration to permit imaging of the target organ or tissue without a disturbing background activity, for example, due to detached label. Further it is of utmost importance, that the compounds and compositions of the present invention are also well suitable for detecting and localizing tissues having neurokinine 1 receptors, when these tissues are present in the abdominal region of the living being, e.g. for detecting and localizing certain tumors in the abdominal cavity and for visualizing Crohn's disease. As will become apparent from the Examples, viz. the liver perfusion model experiments described therein, the tested labelled peptides of the present invention show such a slow liver metabolic clearance, that only a small background activity will result, not only in the abdominal region but also in the circulation system. These metabolic properties, as determined by the model experiments described above and illustrated in the Examples, make the labelled peptide particularly suitable for detecting and localizing tissues having neurokinine 1 receptors, because a favourable target tissue to background ratio may be expected. On the contrary, ¹²⁵I-Bolton Hunter modified SP, known from the above-mentioned in vitro binding studies, shows a relatively fast liver metabolic clearance, which makes this substance significantly less suitable for in vivo application; this will be clear from the appended Examples.

It is another object of the invention to provide compounds and compositions for the therapeutic treatment of tumors having on their surface neurokinine 1 receptors in the body of a warm-blooded living being.

This object can be achieved according to a different aspect of the present invention by providing a composition for administration to said being which comprises, in a quantity effective for combatting or controlling tumors, a small peptide as defined above, wherein said peptide has been labelled via a suitable linker, as mentioned above, with an isotope suitable for the purpose in view. Suitable isotopes for combatting or controlling tumors are beta-emitting isotopes such as Re-186, Re-188, As-77, Y-90, Cu-67, Er-169, Sn-121, Te-127, Pr-142, Pr-143, Au-198, Pd-109 and Dy-165.

The selective affinity of the above labelled peptides to neurokinine 1 receptors make these labelled compounds particularly suitable for therapeutic treatment of certain malignant tumors that are related to neurokinine 1 binding places, such as malignant glioma, pheochromocytoma, paraganglia and SCLC.

The labelled peptide of the invention is derived from a compound of the general formula

R₁-(A₁)ₘ-A₂)ₙ-A₃)ₒ-Pro)ₚ-A₄)_{q}-A₅-Phe-A₆-A₇-A₈-Met(O)ₛ-R₂ (I)

wherein all of the symbols m, n, o, p and q are 1, or all but one of the symbols m, n, o, p and q are 1 and the remaining symbol is 0;
R₁ is a hydrogen atom or a C₁-C₄ alkylcarbonyl group;
R₂ is an amino group, a hydroxy group or a C₁-C₄ alkoxy group;
A₁ is Arg, Gly or 5-oxo-Pro (pGlu);
A₂ is Pro or β- Ala;
A₃ is Lys or Asp;
A₄ is Gln, Asn or 5-oxo-Pro;
A₅ is Gln, Lys, Arg, N-acylated Arg or 5-oxo-Pro;
   or wherein A₅ together with A₃ forms a cystine moiety;
A₆ is Phe or Tyr;
A₇ is Gly, Sar or Pro;
A₈ is Leu or Pro; and
s is 0, 1 or 2;
or a Tyr^{o} derivative thereof.

Suitable examples of such a compound of the above general formula I are:
(1) H-Arg-Pro-Lys-Pro-Gln-Gln-Phe-Phe-Gly-Leu-Met-NH₂ (substance P).
(2) H-Arg-Pro-Lys-Pro-Gln-Gln-Phe-Phe-Sar-Leu-Met(O₂)-NH₂,
(3) H- -Ala-Gln-Gln-Phe-Phe-Sar-Leu-Met(O₂)-NH₂,
(4) H-Arg-Pro-Lys-Pro-Gln-Gln-Phe-Tyr-Gly-Leu-Met-NH₂ and 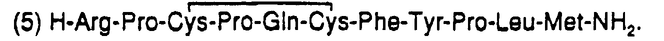
and their Tyr⁰ derivatives.

The invention also relates to the use of labelled small peptides as defined above, which are composed of amino acids, at least one of which has the d-configuration. The labelled peptides to be used according to the invention may also comprise so-called pseudo peptide bonds, viz. -CH₂-NH- bonds, in addition to the natural amide bonds, viz. -CO-NH- bonds.

The desired isotope as defined above should be firmly attached to the small peptide molecule to reduce detachment of this label after administration to the living being. As will be clear from the above, the proper choice of the linker is an essential aspect of the present invention. The small peptide can be labelled with the desired halogen radioisotope directly or indirectly, viz. via a tyrosyl group. Direct labelling may be carried out, for example, by introducing a halogen atom or radioactive halogen atom into an activated aromatic group (e.g. tyrosyl or imidazolyl) present in the peptide molecule in a manner known per se, if desired followed by exchange with 1-123, 1-131, Br-75 or Br-76, e.g. by the method as described in European Patent 165630. In general, however, labelling via said linker is preferred, said linker being capable of reacting with an amino group, preferably a terminal amino group, of said peptide, and having a functional group for binding said isotope. By using said linker, the desired isotope can generally better be introduced into the peptide molecule. It is of advantage to attach the linker to a terminal amino group of the peptide molecule, in order to maintain the biological properties of this peptide as much as possible. It has been found that a tyrosyl linking group is very suitable for producing a radioactive-halogen-labelled peptide, which combines a selective affinity to NK1 receptors with a slow liver metabolic clearance. A tyrosyl moiety can be introduced into the amino acid chain, preferably in the 0-position, during the peptide synthesis; alternatively, the tyrosyl group can be introduced by a separate reaction of the peptide with tyrosine or a functional derivative thereof. The derivatized peptide, thus obtained, is substituted by the desired halogen radioisotope by an appropriate reaction. In this manner the peptide can be labelled with the desired radioactive halogen isotope without affecting its biological properties. The radiohalogenating reaction is preferably performed by reacting the peptide with a solution of an alkali metal radionuclide selected from ¹²³I⁻, ¹³¹I⁻, ⁷⁵Br⁻ and ⁷⁶Br⁻ under the influence of a halide-oxidizing agent, such as chloramine T or iodogen. Alternatively, the above substitution reaction can be carried out with non-radioactive halogen, after which halo-exchange with radioactive halogen is performed, e.g. as described in European patent 165630.

A suitable linker for attaching a metal isotope to the small peptide is provided with a chelating group. Such isotopes are selected from the group consisting of Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, Re-186, Re-188, As-77, Y-90, Cu-67, Er-169, Sn-121, Te-127, Pr-142, Pr-143, Au-198, Pd-109 and Dy-165. Various coupling agents for attaching metal isotopes to proteins are described in literature, such as compounds which after coupling with the protein can complex the metal isotope by an N₂S₂-, N₃S- or N₄-tetradentate ring structure, amino-containing compounds such as the maleimide derivatives disclosed in European patent application 178125, peptide-derivatives, and compounds comprising chelating groups such as isocyanate, formyl, diazonium, isothiocyanate, or alkoxycarbimidoyl groups. Suitable linkers are derived from N-containing di- or polyacetic acids or their derivatives, namely ethylene diaminetetraacetic acid (EDTA), diethylene triamine pentaacetic acid (DTPA), ethylene glycol-0,0'-bis(2-aminoethyl)-N,N,N',N'-tetraacetic acid (EGTA), N,N'-bis(hydroxybenzyl)ethylenediamine-N,N'-diacetic acid (HBED), triethylenetetramine hexaacetic acid (TTHA), substituted EDTA or -DTPA, 1,4,7,10-tetra-azacyclododecane-N,N',N",N"'-tetraacetic acid (DOTA) or 1,4,8,11-tetraazacyclotetradecane-N,N',N",N"'-tetraacetic acid (TETA). For modifying the small peptide in question, however, a linker is preferred which is derived from the coupling agents described in PCT application WO 89/07456. These coupling agents are generally be represented by the formula 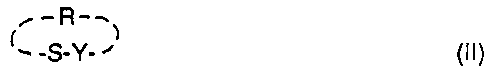 wherein
R is a branched or non-branched, optionally substituted hydrocarbyl radical, which may be interrupted by one or more hetero-atoms selected from N, O and S and/or by one or more NH groups, and
Y is a group which is capable of reacting with an amino group of the peptide and which is preferably selected from the group consisting of carbonyl, carbimidoyl, N-(C₁-C₆)alkylcarbimidoyl, N-hydroxycarbimidoyl and N-(C₁-C₆)alkoxycarbimidoyl. Examples of coupling agents described therein are unsubstituted or substituted 2-iminothiolanes and 2-iminothiacyclohexanes.

The modification of the peptide in question, i.e. the reaction with the coupling agent, results in a peptide conjugate. This reaction can generally be carried out in a simple manner. In the subsequent complex-forming reaction, the metal isotope is presented to the peptide conjugate in the form of a salt or chelate. In the latter case relatively weak chelators are used, e.g. a phosphonate or polyphosphonate, an oxinate, a carboxylate, a hydroxycarboxylate, an aminocarboxylate or an enolate. Then the desired complex is formed by ligand exchange. The complex forming reactions can generally be carried out in a simple manner and under conditions which spare the peptide.

The invention further relates to a pharmaceutical composition to be used for detecting and localizing tissues having neurokinine 1 receptors in the body of a warm-blooded living being, which composition comprises in addition to a pharmaceutically acceptable carrier and, if desired, at least one pharmaceutically acceptable adjuvant, as the active substance a small peptide having a selective affinity to neurokinine 1 receptors, said peptide being labelled with a detectable isotope as defined above. Such a composition is intended for diagnostic application, or, if labelled with a suitable isotope as indicated hereinbefore, for therapeutic application. If desired, the composition so obtained can be brought into a form more suitable for intravenous or subcutaneous application, e.g. by adding a pharmaceutically acceptable liquid carrier material. For intravenous or subcutaneous application the solution should of course be in a sterile condition.

The invention also relates to a labelled small peptide to be used as an active ingredient in the composition as described above, said peptide having a selective affinity to neurokinine 1 receptors and being labelled with an isotope as defined hereinbefore.

In case a radioactive labelled peptide is used as a diagnostic agent, it is frequently impossible to put the ready-for-use composition at the disposal of the user, in connection with the often poor shelf life of the radiolabelled compound and/or the short half-life of the radionuclide used. In such cases the user will carry out the labelling reaction with the radionuclide in the clinical hospital or laboratory. For this purpose the various reaction ingredients are then offered to the user in the form of a so-called "kit". It will be obvious that the manipulations necessary to perform the desired reaction should be as simple as possible to enable the user to prepare from the kit the radioactive labelled composition by using the facilities that are at his disposal. Therefore the invention also relates to a kit for preparing a radiopharmaceutical composition.

Such a kit according to the present invention may comprise (i) a small peptide having a selective affinity to neurokinine 1 receptors and optionally provided with a linking group as defined above, to which substance, if desired, an inert pharmaceutically acceptable carrier and/or formulating agents and/or adjuvants is/are added, (ii) a solution of a compound of a suitable radionuclide, and (iii) instructions for use with a prescription for reacting the ingredients present in the kit.

Suitable radionuclides for the above kit are: Pb-203, Ga-67, Ga-68, As-72, In-111, In-113m, Ru-97, Tc-99m, Re-186, Re-188, Cu-62, Cu-64, Fe-52, Mn-52m, Cr-51, I-123, I-131, Br-75, Br-76, As-77, Y-90, Cu-67, Er-169, Sn-121, Te-127, Pr-142, Pr-143, Au-198, Pd-109 and Dy-165. If in such a kit the radionuclide is a radioactive halogen selected from 1-123, 1-131, Br-75 and Br-76, preferably an alkali metal halogenide, as generally known in the art, is used as an ingredient of the kit, if desired, accompanied by a halide oxidation agent, such as chloramine T or iodogen^{R}. Preferably the small peptide to be used as an ingredient of the above kit has been modified by a reaction with a coupling agent as defined hereinbefore. The resulting peptide conjugate provides a facility for firmly attaching the radionuclide in a simple manner. Suitable coupling agents for modifying the peptide are described in detail hereinbefore. N-containing di-or polyacetic acids or their derivatives, such as the compounds mentioned before, have proved to be pre-eminently suitable for attaching various metal radionuclides, such as In-111 and In-113m, to the peptide molecules. The kit to be supplied to the user may also comprise the ingredient(s) defined sub (i) above, together with instructions for use, whereas the solution of a compound of the radionuclide, defined sub (ii) above, which solution has a limited shelf life, may be put to the disposal of the user separately.

In case the kit serves to prepare a radiopharmaceutical composition labelled with Tc-99m, Re-186 or Re-188, such a kit according to the present invention may comprise, in addition to the ingredient(s) defined sub (i) above, (ii) a reducing agent and, if desired, a chelator, and (iii) instructions for use with a prescription for reacting the ingredients of the kit with Tc-99m in the form of a pertechnetate solution, or with Re-186 or Re-188 in the form of a perrhenate solution. If desired, the ingredients of the kit may be combined, provided they are compatible. The kit should comprise a reducing agent to reduce the pertechnetate or perrhenate, for example, a dithionite, a metallic reducing agent or a complex-stabilizing reducing agent, e.g. SnCl₂, Sn(II)-tartrate, Sn(ll)-phosphonate or -pyrophosphate, or Sn(II)-glucoheptonate. The pertechnetate or perrhenate solution can simply be obtained by the user from a suitable generator.

In a preferred embodiment the kit according to the present invention comprises, a modified peptide or a peptide conjugate, obtained by modifying the peptide as defined hereinbefore by a treatment with a coupling agent. Suitable coupling agents have been described hereinbefore. The use of a compound of the general formula 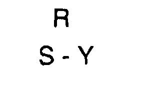 wherein the symbols have the meanings given hereinbefore, as a coupling agent is to be preferred.

When the radionuclide is present in the kit itself, the complex forming reaction with the peptide conjugate can simply be produced by combining the components in a neutral medium and causing them to react. For that purpose the radionuclide may be presented to the peptide conjugate in the form of a chelate bonded to a comparatively weak chelator, as described hereinbefore.

When the kit comprises a peptide conjugate as defined hereinbefore and is intended for the preparation of a radiopharmaceutical composition, labelled with Tc-99m, Re-186 or Re-188, the radionuclide will preferably be added separately in the form of a pertechnetate or perrhenate solution. In that case the kit will comprise a suitable reducing agent and, if desired, a chelator, the former to reduce the pertechnetate or the perrhenate. As a reducing agent may be used, for example, a dithionite ora metallic reducing agent. The ingredients may optionally be combined, provided they are compatible. Such a monocomponent kit, in which the combined ingredients are preferably lyophilized, is excellently suitable for being reacted, by the user, with the radionuclide solution. As a reducing agent for the above-mentioned kits is preferably used a metallic reducing agent, for example, Sn(II), Fe(II), Cu(I), Ti(III) or Sb(III); Sn(II) is excellently suitable. The peptide constituent of the above-mentioned kits, i.e. preferably the peptide conjugate, may be supplied as a solution, for example, in the form of a physiological saline solution, or in some buffer solution, but is preferably present in a dry condition, for example, in the lyophilized condition. When used as a component for an injection liquid it should be sterile, in which, when the constituent is in the dry state, the user should preferably use a sterile physiological saline solution as a solvent. If desired, the above-mentioned constituent may be stabilized in the conventional manner with suitable stabilizers, for example, ascorbic acid, gentisic acid or salts of these acids, or it may comprise other auxiliary agents, for example, fillers, such as glucose, lactose, or mannitol.

The invention will now be described in greater detail with reference to the ensuing specific Examples.

### EXAMPLE I

### Synthesis of labelled peptides

Tyr⁰-substance P is synthetized according to the solid-phase Merrifield method (J. Am. Chem. Soc. 85, 1963, 2149), using the so-called Fmoc-strategy (Int. J. Pept. Protein Res. 35, 1990, 161-214). This means that Fmoc-protected amino acids are successively coupled, each time followed by cleavage of the protecting Fmoc-group in basic medium.

DTPA-substance P is prepared by the method described by Hnatowich (US Patent 4,479,930) using lysine-protected substance P and DTPA-dianhydride. In a corresponding manner DTPA-Tyr⁰-substance P is prepared.

Tyr⁰-substance P is labelled with 1-123 by dissolving this compound in phosphate buffer and adding an equimolar quantity of ¹²³I⁻-sodium iodide to this solution in the presence of chloramine T. The labelling of the DTPA-modified peptides with In-111 is performed by dissolving said peptides in 0.01M acetic acid and mixing these solutions with ¹¹¹In-InCl₃ solution in 0.5M aqueous sodium acetate at room temperature. Then HEPES buffer is added for neutralizing purposes.

### EXAMPLE II

### Binding studies

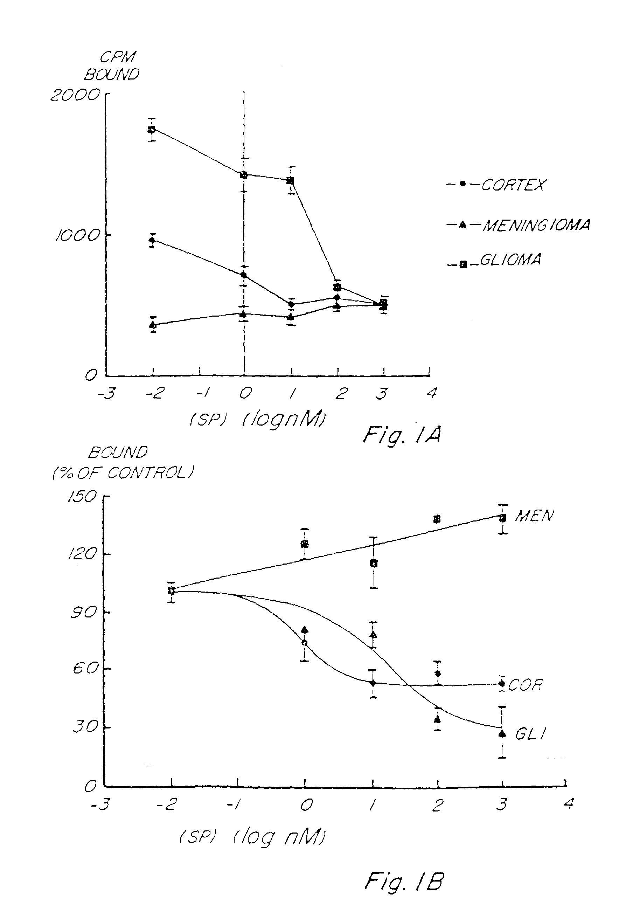

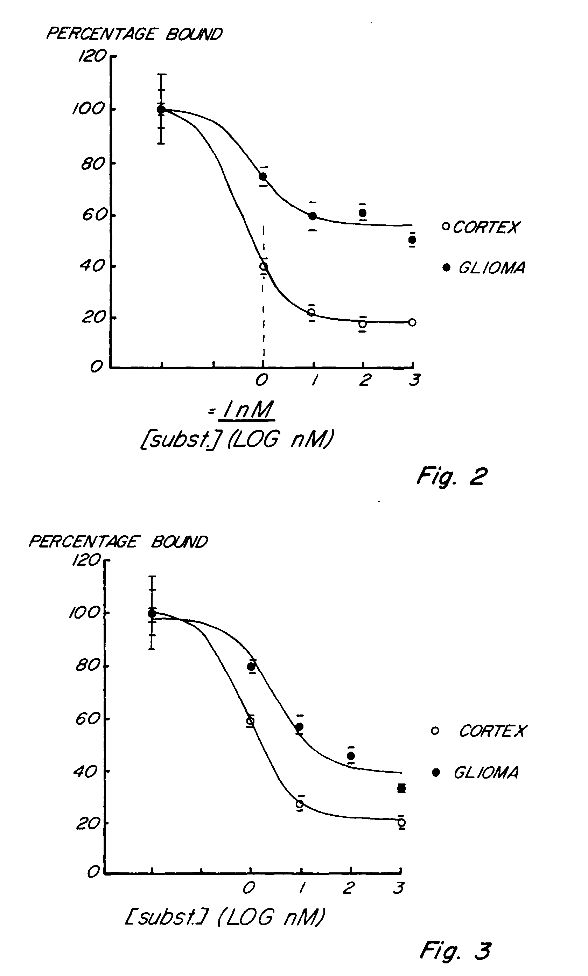

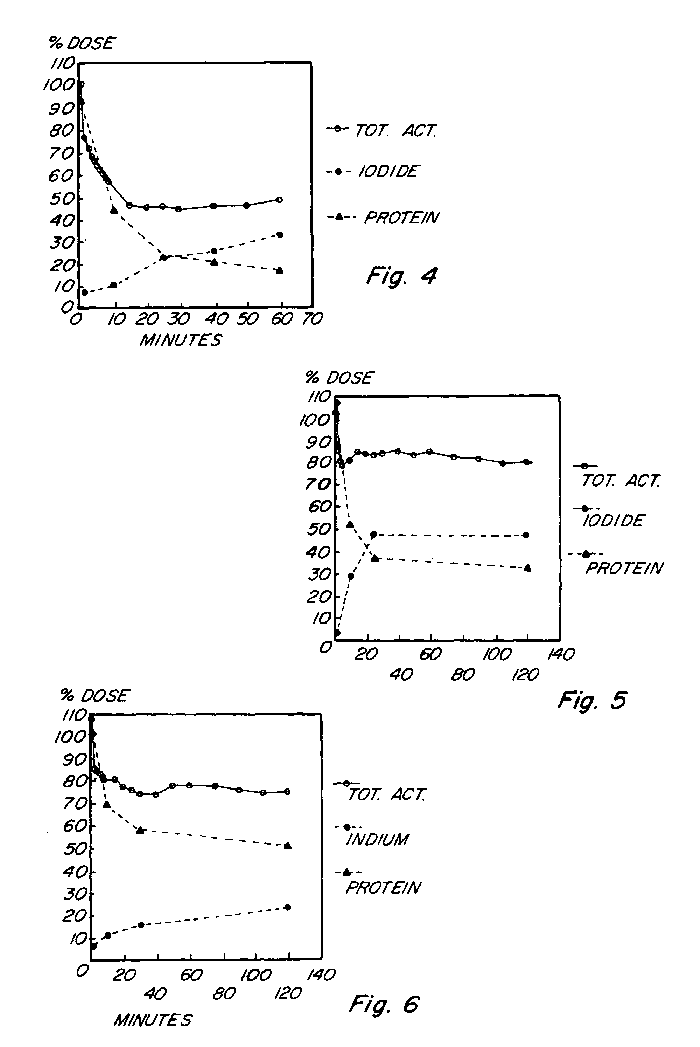

Binding studies in vitro are carried out in a standardized system by studying the displacement of ¹²⁵I-Bolton Hunter substance P {[¹²⁵I]BH-SP} by the above-synthetized labelled peptides. Figure 1 shows that [¹²⁵I]BH-SP specifically binds to membranes of the cortex of rat brains and to human glioma membranes, but not to human meningioma membranes.
The displacement of [¹²⁵I]BH-SP by iodinated Tyr⁰-substance P is demonstrated by Figure 2; the measured IC₅₀ value (nM) is 0.4 (cortex) and 0.7 (glioma). The displacement by In-complexed DTPA-Tyr⁰-substance P is demonstrated by Figure 3; the IC₅₀ value (nM) is 0.9 (cortex) and 3.2(glioma).

The results show that the labelled peptides of the invention bind specifically to the SP-receptors, present on the tissue membranes, because they are able to displace SP from these receptors.

### EXAMPLE III

### Liver perfusion experiments with labelled substance P

A liver perfusion model is used as described by Docter et al. in Endocrinology 1990, 126, 451-459. It is a generally accepted fact in this art, that handling of bio-active substances by the isolated perfused rat liver according to this model has a very good predictable value for the appearance of metabolites of the used substance in the circulation of human beings. In short, in this model isolated livers are perfused in a recirculating system with Krebs-Ringer medium supplemented with 1% bovine serum albumin and 10 mM glucose. After 0.5 hr preperfusion, experiments are started by addition of the labelled substance P (5 µCi) to the medium. At regular time points medium samples are taken. Medium samples are analyzed by column chromatography, using Seppak^{(R)} C-18 columns.

The average results are presented in Figures 4-6, showing the disappearance of the total radioactivity ("tot. act.") and of the peptide-bound radioactivity ("protein") with concomitant appearance of non-peptide bound radioactivity in the circulating medium as a degradation product.

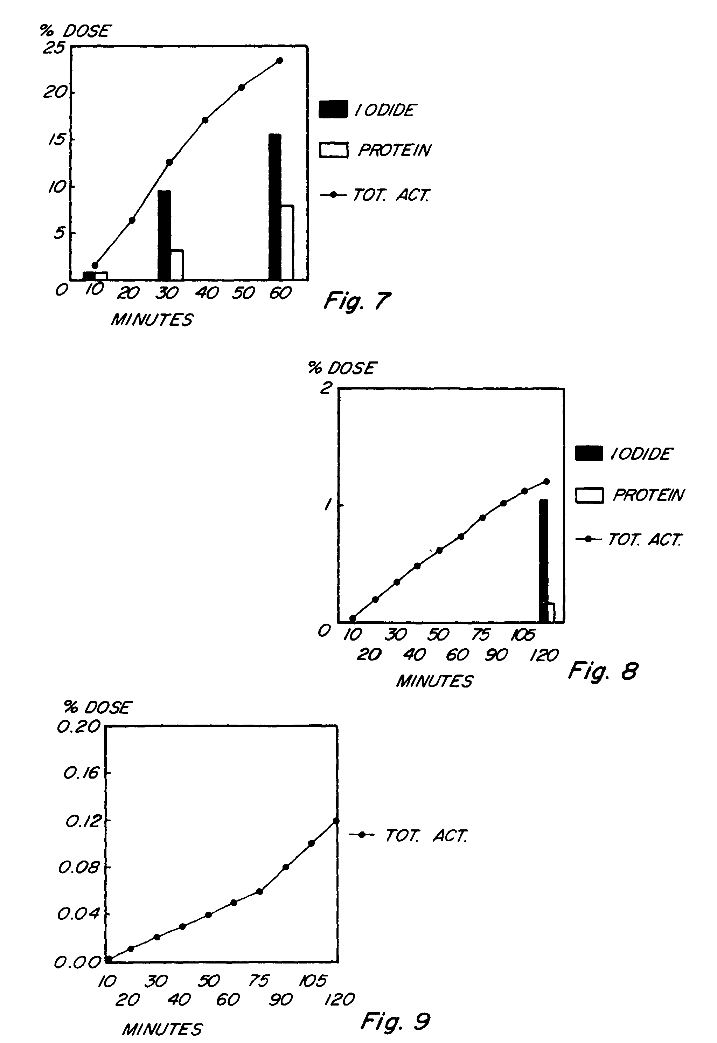

Figures 7-9 show the excretion of the tested labelled peptides into the bile.

In Figures 4 and 7 the results are presented of [¹²⁵I]BH-SP, showing that after 60 minutes, approximately 50% of the total radioactivity has disappeared, apparently to a considerable extent via liver metabolic clearance: approximately 25% of radioactivity is excreted into the bile. The differences with the tested labelled peptides of the invention are striking. The results of [¹²³I]Tyr⁰-SP are presented in Figures 5 and 8, showing that after 60 minutes only about 15% of the total radioactivity has disappeared; the radioactivity excreted into the bile is only about 0.8%, rising to only about 1.2% after 120 minutes. Even more favourable results are obtained with [¹¹¹In]DTPA-Tyr⁰-SP: Figures 6 and 9; only a negligible excretion of radioactivity into the bile can be observed after 120 minutes.

From the above results can be concluded that, as opposed to [¹²³I]BH-SP, the labelled peptides according to the invention are promising substances for scintigraphic imaging purposes with regard to handling by the liver. It can be concluded, that the metabolic clearance of these substances by the liver is negligible, resulting in a low background activity.

### EXAMPLE IV

### In vivo experiments

A pilot experiment with [¹¹¹In]DTPA-Tyr⁰-SP is carried out in rats wherein granulomas are invoked. It is well-known that granulomas contain SP-receptors. The radiolabelled peptide is injected in a quantity corresponding to 500 µCi. A granuloma evoked in a leg of the rats can easily be visualized by scanning 2.5 hours after injection. The rats are sacrificed at four hours or 24 hours after injection, and various organs, including the granulomas, are excised and weighed. The measured radioactivity, corrected for the weight of the organs, is presented in Table 1 below. In Table 1, the radioactivity is expressed in ratios compared to the radioactivity in the blood, arbitrarily fixed at 1.0: relative radioactivity.

From the results it can be concluded, that a considerable accumulation of radioactivity, sufficiently for scanning purposes, takes place in the granulomas, and that the liver metabolic clearance is negligible in comparison with the renal clearance.

**TABLE 1**

| Relative Radioactivity | | | |
|---|---|---|---|
| | 4 h a.i. | 24 h a.i. | |
| ORGAN | Rat 1 | Rat 2 | Rat 3 |
| granuloma | 4.6 | 4.1 | 3.8 |
| kidneys | 195.2 | 295.8 | 309.6 |
| liver | 2.7 | 8.0 | 3.7 |
| blood | 1.0 | 1.0 | 1.0 |

## Claims

1. A labelled peptide having a selective affinity to neurokinine 1 receptors, wherein said peptide is derived from a compound of the general formula
R₁-(A₁)ₘ-A₂)ₙ-A₃)ₒ-Pro)ₚ-A₄)_{q}-A₅-Phe-A₆-A₇-A₈-Met(O)ₛ-R₂
wherein all of the symbols m, n, o, p and q are 1, or all but one of the symbols m, n, o, p and q are 1 and the remaining symbol is zero;
R₁ is a hydrogen atom or a C₁-C₄ alkylcarbonyl group;
R₂ is an amino group, a hydroxy group or a C₁-C₄ alkoxy group;
A₁ is Arg, Gly or 5-oxo-Pro (pGlu);
A₂ is Pro or β-Ala;
A₃ is Lys or Asp;
A₄ is Gln, Asn or 5-oxo-Pro;
A₅ is Gln, Lys, Arg, N-acylated Arg or 5-oxo-Pro; or
wherein
A₅ together with A₃ forms a cystine moiety;
A₆ is Phe or Tyr;
A₇ is Gly, Sar or Pro;
A₈ is Leu or Pro; and
S is zero, 1 or 2;
or a Tyr^{o} derivative thereof;
wherein said peptide is labelled with (a) a metal isotope selected from Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, Re-186, Re-188, As-77, Y-90, Cu-67, Er-169, Sn-121, Te-127, Pr-142, Pr-143, Au-198, Pd-109 and Dy-165, said metal isotope being attached to said peptide via a linker capable of reacting with an amino group of said peptide, and having a chelating group for chelating said metal isotope, and derived from ethylene diamine tetraacetic acid (EDTA), diethylene triamine pentaacetic acid (DPTA), ethylene glycol-O,O'-bis(2-aminoethyl)-N,N,N', N'-tetraacetic acid (EGTA), N,N'-bis(hydroxybenzyl) ethylenediamine-N,N'-diacetic acid (HBED), triethylenetetramine hexaacetic acid (TTHA), substituted EDTA or substituted DTPA, 1,4,7,10-tetra-azacyclododecane-N, N', N'', N'''-tetraacetic acid (DOTA) or 1,4,8,11-tetraazacyclotetradecane-N, N',N'',N'''-tetraacetic acid (TETA) or via a linker derived from a compound of the general formula 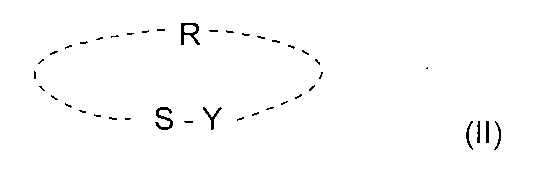 wherein R is a branched or non-branched, optionally substituted hydrocarbyl radical, which may be interrupted by one or more hetero-atoms selected from N, O and S and/or by one or more NH groups, and Y is a group which is capable of reacting with an amino group of the peptide and which is preferably selected from carbonyl, carbimidoyl, N-(C₁-C₆)-alkylcarbimidoyl, N-hydroxycarbimidoyl and N-(C₁-C₆) alkoxycarbimidoyl; or with (b) a detectable halogen radioisotope selected from I-123, I-131, Br-75 and Br-76, said halogen radioisotope being attached to said peptide directly or via a linking tyrosyl group.

2. A compound according to claim 1, for detecting and localizing tissues having neurokinine 1 receptors in the body of a warm-blooded living being, wherein said peptide is labelled with (a) a detectable metal isotope selected from Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, or with (b) a detectable halogen radioisotope selected from I-123, I-131, Br-75 and Br-76.

3. A compound as claimed in claim 1 or claim 2, wherein said detectable halogen radioisotope is attached to a terminal amino group of said peptide via a tyrosyl linking group.

4. A compound according to claim 1, for the therapeutic treatment of tumors having on their surface neurokinine 1 receptors in the body of a warm-blooded living being, wherein said peptide is labelled with a metal isotope selected from Re-186, Re-188, As-77, Y-90, Cu-67, Er-169, Sn-121, Te-127, Pr-142, Pr-143, Au-198, Pd-109 and Dy-165.

5. A pharmaceutical composition comprising in addition to a pharmaceutically acceptable carrier and, if desired, at least one pharmaceutically acceptable adjuvant, as the active substance a compound according to any one of claims 1 to 4.

6. Use of a compound according to claim 2 for the manufacture of a diagnostic agent for detecting and localizing tissues having neurokinine 1 receptors in the body of a warm-blooded living being.

7. Use of a compound according to claim 4 for the manufacture of a therapeutic agent for treating tumors having on their surface neurokinine 1 receptors in the body of a warm-blooded living being.

8. A kit for preparing a radiopharmaceutical composition, comprising (i) a small peptide having a selective affinity to neurokinine 1 receptors and optionally provided with a linking group as defined in claim 1, to which substance, if desired, an inert pharmaceutically acceptable carrier and/or formulating agents and/or adjuvants is/are added, (ii) a solution of a compound of a radionuclide selected from the group consisting of Pb-203, Ga-67, Ga-68, As-72, In-111, In-113m, Tc-99m, Re-186, Re-188, Ru-97, Cu-62, Cu-64, Fe-52, Mn-52m, Cr-51, I-123, I-131, Br-75, Br-76, As-77, Y-90, Cu-67, Er-169, Sn-121, Te-127, Pr-142, Pr-143, Au-198, Pd-109 and Dy-165, and (iii) instructions for use with a prescription for reacting the ingredients present in the kit.

9. A kit for preparing a radiopharmaceutical composition, comprising (i) a small peptide having a selective affinity to neurokinine 1 receptors and optionally provided with a linking group as defined in claim 1, to which substance, if desired, an inert pharmaceutically acceptable carrier and/or formulating agents and/or adjuvants is/are added, (ii) a reducing agent and, if desired, a chelator, said ingredients (i) and (ii) optionally being combined, and (iii) instructions for use with a prescription for reacting the ingredients of the kit with Tc-99m in the form of a pertechnetate solution or with Re-186 or Re-188 in the form of a perrhenate solution.

10. A kit according to claim 8 or claim 9, comprising a peptide conjugate, obtained by modifying a small peptide, as defined in claim 1, by a reaction with a linker having a functional group for binding or chelating a radionuclide as defined in claim 1.

11. A kit according to claim 10, comprising a peptide conjugate obtained by modifying said small peptide by a reaction with a linker, said linker being derived from ethylene diaminetetraacetic acid (EDTA), diethylene triamine pentaacetic acid (DTPA), ethylene glycol-O,O'-bis(2-aminoethyl)-N,N,N',N'-tetraacetic acid (EGTA), N,N'-bis(hydroxybenzyl)ethylenediamine-N,N'diacetic acid (HBED), triethylenetetramine hexaacetic acid (TTHA), substituted EDTA or -DTPA, 1,4,7,10-tetra-azacyclododecane-N,N', N",N"'-tetraacetic acid (DOTA) or 1,4,8,11-tetraazacyclotetradecane-N,N',N",N"'-tetraacetic acid (TETA), or from a compound of the general formula 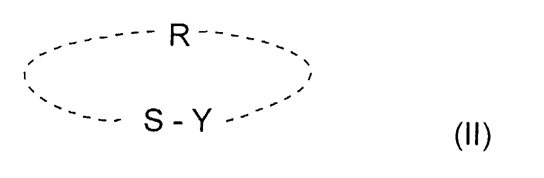 wherein
R is a branched or non-branched, optionally substituted hydrocarbyl radical, which may be interrupted by one or more hetero-atoms selected from N, O and S and/or by one or more NH groups, and
Y is a group which is capable of reacting with an amino group of the peptide and which is preferably selected from the group consisting of carbonyl, carbimidoyl, N-(C₁-C₆)-alkylcarbimidoyl, N-hydroxycarbimidoyl and N-(C₁-C₆)-alkoxycarbimidoyl.

## Patentansprüche

1. Peptid mit einer selektiven Affinität zu Neurokinin-1-Rezeptoren, das von einer Verbindung der allgemeinen Formel
R₁-(A₁)ₘ-A₂)ₙ-A₃)ₒ-Pro)ₚ-A₄)_{q}-A₅-Phe-A₆-A₇-A₈-Met(O)ₛ-R₂
abgeleitet ist, worin alle Symbole m, n, o, p und q die Zahl 1 sind oder alle Symbole m, n, o, p und q bis auf eins die Zahl 1 sind und das übrige Symbol null ist,
R₁ ein Wasserstoffatom oder eine C₁-C₄-Alkycarbonylgruppe ist,
R₂ eine Aminogruppe, eine Hydroxygruppe oder eine C₁-C₄-Alkoxygruppe ist,
A₁ Arg, Gly oder 5-Oxo-Pro (pGlu) ist,
A₂ Pro oder β-Ala ist,
A₃ Lys oder Asp ist,
A₄ Gln, Asn oder 5-Oxo-Pro ist,
A₅ Gln, Lys, Arg, N-acetyliertes Arg oder 5-Oxo-Pro ist,
oder worin
A₅ zusammen mit A₃ eine Cystin-Gruppe bildet,
A₆ Phe oder Tyr ist,
A₇ Gly, Sar oder Pro ist,
A₈ Leu oder Pro ist, und
S null, 1 oder 2 ist,
oder ein Tyr^{o}-Derivat davon,
wobei das genannte Peptid markiert ist mit (a) einem unter Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, Re-186, Re-188, As-77, Y-90, Cu-67, Er-169, Sn-121, Te-127, Pr-142, Pr-143, Au-198, Pd-109 und Dy-165 ausgewählten Metallisotop, das an das genannte Peptid über ein Verbindungsglied gebunden ist, das zur Umsetzung mit einer Aminogruppe des Peptids befähigt ist und ein chelatbildende Gruppe zur Chelatbildung mit dem genannten Metallisotop hat und sich ableitet von Ethylendiamintetraessigsäure (EDTA), Diethylentriaminpentaessigsäure (DPTA), Ethylenglykol-O,O'-bis(2-aminoethyl)-N,N,N',N'-tetraessigsäure (EGTA), N,N'-bis(Hydroxybenzyl)ethylendiamin-N,N'-dieessigsäure (HBED), Triethylentetraminhexaessigsäure (TTHA), substituierter EDTA oder substituierter DTPA, 1,4,7,10-Tetraazazyklododekan-N,N',N'',N'''-tetraessigsäure (DOTA) oder 1,4,8,11-Tetraazazyklotetradekan-N,N',N'',N'''-tetraessigsäure (TETA), oder über ein Verbindungsglied, das sich von einer Verbindung der allgemeinen Formel 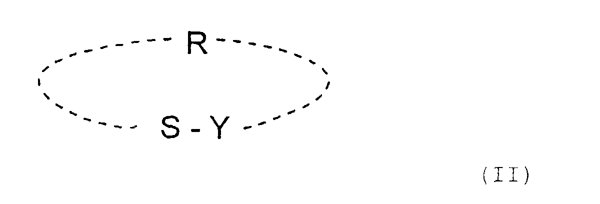 ableitet, worin R ein verzweigter oder unverzweigter, wahlweise substituierter Kohlenwasserstoffrest ist, der durch ein oder mehrere, unter N, O und S ausgewählte Heteroatome und/oder durch eine oder mehrere NH-Gruppen unterbrochen sein kann, und Y eine Gruppe ist, die zur Umsetzung mit einer Aminogruppe des Peptids befähigt ist und vorzugsweise unter Carbonyl, Carbimidoyl, N-(C₁-C₆)-Alkylcarbimidoyl, N-Hydroxycarbimidoyl und N-(C₁-C₆)-Alkoxycarbimidoyl ausgewählt ist; oder markiert ist mit (b) einem nachweisbaren Halogenisotop, das unter I-123, I-131, Br-75 und Br-76 ausgewählt ist und direkt oder über eine verbindende Tyrosylgruppe an dem genannten Peptid hängt.

2. Verbindung nach Anspruch 1 zum Nachweis und zur Lokalisierung von Geweben mit Neurokinin-1-Rezeptoren in dem Körper eines Warmblüters, wobei das Peptid markiert ist mit (a) einem nachweisbaren Metallisotop, das unter Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56 ausgewählt ist, oder mit (b) einem nachweisbaren Halogenisotop, das unter I-123, I-131, Br-75 und Br-76 ausgewählt ist.

3. Verbindung nach Anspruch 1 oder Anspruch 2, in der das nachweisbare Halogenisotop über eine Tyrosyl-Verbindungsgruppe an einer endständigen Aminogruppe des Peptids hängt.

4. Verbindung nach Aspruch 1 zur therapeutischen Behandlung von Tumoren mit Neurokinin-1-Rezeptoren auf ihrer Oberfläche in dem Körper eines Warmblüters, wobei das Peptid mit einem Metallisotop markiert ist, daß unter Re-186, Re-188, As-77, Y-90, Cu-67, Er-169, Sn-121, Te-127, Pr-142, Pr-143, Au-198, Pd-109 und Dy-165 ausgewählt ist.

5. Pharmazeutische Zusammensetzung, die neben einem pharmazeutisch zulässigen Träger und gewünschtenfalls wenigsten einem pharmazeutisch zulässigen Hilfsstoff eine Verbindung nach einem der Ansprüche 1 bis 4 als aktive Substanz enthält.

6. Verwendung einer Verbindung nach Anspruch 2 für die Herstellung eines diagnostischen Mittels zum Nachweis und zur Lokalisierung von Geweben mit Neurokinin-1-Rezeptoren in dem Körper eines Warmblüters

7. Verwendung einer Verbindung nach Anspruch 4 zur Herstellung eines therapeutischen Mittels zur Behandlung von Tumoren mit Neurokinin-1-Rezeptoren auf ihrer Oberfläche in dem Körper eines Warmblüters.

8. Präparatesatz zur Herstellung einer pharmazeutischen Zusammensetzung mit (i) einem kleinen Peptid mit selektiver Affinität zur Neurokinin-1-Rezeptoren und wahlweise mit einer Verbindungsgruppe gemäß Definition in Anspruch 1, welcher Substanz gewünschtenfalls ein inerter pharmazeutisch zulässiger Träger und/oder Formulierungsmittel und/oder Begleitstoffe zugesetzt sind, (ii) einer Lösung einer Verbindung eines Radionuklids, das aus der aus Pb-203, Ga-67, Ga-68, As-72, In-111, In-113m, Tc-99m, Re-186, Re-188, Ru-97, Cu-62, Cu-64, Fe-52, Mn-52m, Cr-51, I-123, I-131, Br-75, Br-76, As-77, Y-90, Cu-67, Er-169, Sn-121, Te-127, Pr-142, Pr-143, Au-198, Pd-109 und Dy-165 bestehenden Gruppe ausgewählt ist, und (iii) Gebrauchsanweisungen mit einer Vorschrift zur Umsetzung der in dem Präparatesatz vorliegenden Bestandteile.

9. Präparatesatz zur Herstellung einer radiopharmazeutischen Zusammensetzung mit (i) einem kleinen Peptid mit selektiver Affinität zur Neurokinin-1-Rezeptoren und wahlweise mit einer Verbindungsgruppe gemäß Definition in Anspruch 1, welcher Substanz gewünschtenfalls ein inerter pharmazeutisch zulässiger Träger und/oder Formulierungsmittel und/oder Begleitstoffe zugesetzt sind, (ii) einem Reduktionsmittel und gewünschtenfalls einem Chelatbildner, wobei die Bestandteile (i) und (ii) wahlweise vereinigt sind, und (iii) Gebrauchsanweisungen mit einer Vorschrift zur Umsetzung der Bestandteile des Präparatesatzes mit Tc-99m in der Form einer Pertechnetat-Lösung oder mit Re-186 oder Re-188 in der Form einer Perrhenat-Lösung.

10. Präparatesatz nach Anspruch 8 oder Anspruch 9 mit einem Peptid-Konjugat, erhalten durch Modifizierung eines kleinen Peptids gemäß Definition in Anspruch 1 durch Umsetzung mit einem Verbindungsglied mit einer funktionellen Gruppe zur Bindung oder Chelatierung eines Radionuklids gemäß Definition in Anspruch 1.

11. Präparatesatz nach Anspruch 10 mit einem Peptidkonjugat, das durch Modifizierung des kleinen Peptids durch Umsetzung mit einem Verbindungsglied erhalten wurde, wobei das Verbindungsglied sich ableitet von Ethylendiamintetraessigsäure (EDTA), Diethylentriaminpentaessigsäure (DPTA), Ethylenglykol-O,O'-bis(2-aminoethyl)-N,N,N',N'-tetraessigsäure (EGTA), N,N'-bis(Hydroxybenzyl)ethylendiamin-N,N'-dieessigsäure (HBED), Triethylentetraminhexaessigsäure (TTHA), substituierter EDTA oder DTPA, 1,4,7,10-Tetraazazyklododekan-N,N',N'',N'''-tetraessigsäure (DOTA) oder 1,4,8,11-Tetraazazyklotetradekan-N,N',N'',N'''-tetraessigsäure (TETA), oder von einer Verbindung der allgemeinen Formel 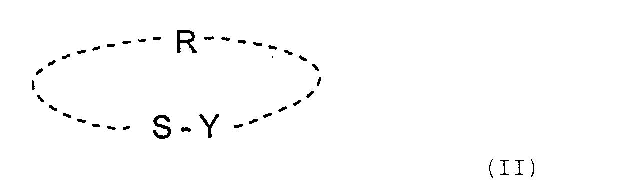 worin
R ein verzweigter oder unverzweigter, wahlweise substituierter Kohlenwasserstoffrest ist, der durch ein oder mehrere unter N, O und S ausgewählte Heteroatome und/oder eine oder mehrere NH-Gruppen unterbrochen sein kann, und
Y eine zur Umsetzung mit einer Aminogruppe des Peptids befähigte Gruppe ist, die vorzugsweise aus der aus Carbonyl, Carbimidoyl, N- (C₁-C₆) -Alkylcarbimidoyl, N-Hydroxycarbimidoyl und N- (C₁-C₆)-Alkoxycarbimidoyl bestehenden Gruppe ausgewählt ist,

## Revendications

1. Peptide marqué ayant une affinité sélective pour les récepteurs de neurokinine 1, ledit peptide étant dérivé d'un composé de formule générale
**R**_{**1**}**-(A**_{**1**}**)**_{**m**}**-A**_{**2**}**)**_{**n**}**-A**_{**3**}**)**_{**o**}**-Pro)**_{**p**}**-A**_{**4**}**)**_{**q**}**-A**_{**5**}**-Phe-A**_{**6**}**-A**_{**7**}**-A**_{**8**}**-Met(O)**_{**s**}**-R**_{**2**}
dans laquelle tous les symboles m, n, o, p et q sont 1, ou tous les symboles m, n, o, p et q, sauf un, sont 1 et le symbole restant est 0 ;
R₁ est un atome d'hydrogène ou un groupe alkylcarbonyle en C₁-C₄ ;
R₂ est un groupe amino, un groupe hydroxy ou un groupe alcoxy en C₁-C₄ ;
A₁ est Arg, Gly ou 5-oxo-Pro (pGlu);
A₂ est Pro ou β-Ala ;
A₃ est Lys ou Asp ;
A₄ est Gln, Asn ou 5-oxo-Pro ;
A₅ est Gln, Lys, Arg, Arg N-acylé ou 5-oxo-Pro ; ou
A₅ forme avec A₃ un fragment cystine ;
A₆ est Phe ou Tyr;
A₇ est Gly, Sar ou Pro ;
A₈ est Leu ou Pro ; et
s est 0,1 ou 2 ;
ou un dérivé Tyr^{o} de ce composé ;
ledit peptide étant marqué par
(a) un isotope de métal choisi parmi Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, Re-186, Re-188, As-77, Y-90, Cu-67, Er-169, Sn-121, Te-127, Pr-142, Pr-143, Au-198, Pd-109 et Dy-165, ledit isotope métallique étant lié audit peptide par l'intermédiaire d'un lieur capable de réagir avec un groupe amino dudit peptide, et ayant un groupe chélatant pour chélater ledit isotope métallique, et dérivé de l'acide éthylènediaminetétracétique (EDTA), de l'acide diéthylènetétraminepentacétique (DPTA), de l'acide éthylèneglycol-O,O'-bis(2-aminoéthyl)-N,N,N',N'-tétracétique (EGTA), de l'acide N,N'-bis(hydroxybenzyl)éthylènediamine-N,N'-diacétique (HBED), de l'acide triéthylènetétraminehexacétique (TTHA), d'EDTA substitué ou de DTPA substitué, de l'acide 1,4,7,10-tétra-azacyclododécane-N,N',N'',N'''-tétracétique (DOTA) ou de l'acide 1,4,8,11-tétra-azacyclotétradécane-N,N',N",N"'-tétracétique (TETA), ou par l'intermédiaire d'un lieur dérivé d'un composé de formule générale 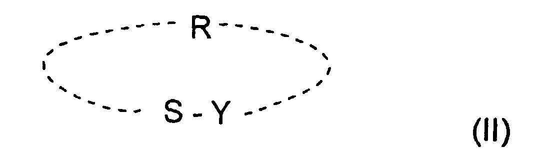 dans laquelle R est un radical hydrocarbyle linéaire ou ramifié, éventuellement substitué, qui peut être interrompu par un ou plusieurs hétéroatomes choisis parmi N, O et S et/ou par un ou plusieurs groupes NH, et Y est un groupe capable de réagir avec un groupe amino du peptide et choisi de préférence parmi les groupes carbonyle, carbimidoyle, N-(alkyl en C₁-C₆)carbimidoyle, N-hydroxycarbimidoyle et N-(alcoxy en C₁-C₆)carbimidoyle ; ou par
(b) un radio-isotope d'halogène décelable choisi parmi I-123, I-131, Br-75 et Br-76, ledit radio-isotope d'halogène étant lié audit peptide directement ou par l'intermédiaire d'un groupe de liaison tyrosyle.

2. Composé selon la revendication 1, pour la détection et la localisation de tissus ayant des récepteurs de neurokinine 1 dans le corps d'un être vivant à sang chaud, ledit peptide étant marqué avec (a) un isotope de métal décelable choisi parmi Tc-99m, Pb-203, Ga-67, Ga-68, As-72, In-111, In-113m, Ru-97, Cu-62, Cu-64, Fe-52, Mn-52m, Cr-51, Na-23, Gd-157, Mn-55, Dy-162, Cr-52, Fe-56, ou avec (b) un radio-isotope d'halogène décelable choisi parmi I-123, I-131, Br-75 et Br-76.

3. Composé selon la revendication 1 ou la revendication 2, dans lequel ledit radio-isotope d'halogène décelable est lié à un groupe amino terminal dudit peptide par l'intermédiaire d'un groupe de liaison tyrosyle.

4. Composé selon la revendication 1, pour le traitement thérapeutique de tumeurs ayant sur la surface des récepteurs de neurokinine 1 dans le corps d'un être vivant à sang chaud, ledit peptide étant marqué avec un isotope de métal choisi parmi Re-186, Re-188, As-77, Y-90, Cu-67, Er-169, Sn-121, Te-127, Pr-142, Pr-143, Au-198, Pd-109 et Dy-165.

5. Composition pharmaceutique comprenant, en plus d'un support pharmaceutiquement acceptable et, si désiré, d'au moins un adjuvant pharmaceutiquement acceptable, un composé selon l'une quelconque des revendications 1 à 4 comme substance active.

6. Utilisation d'un composé selon la revendication 2 pour la fabrication d'un agent de diagnostic pour la détection et la localisation de tissus ayant des récepteurs de neurokinine 1 dans le corps d'un être vivant à sang chaud.

7. Utilisation d'un composé selon la revendication 4 pour la fabrication d'un agent thérapeutique pour le traitement de tumeurs ayant sur la surface des récepteurs de neurokinine 1 dans le corps d'un être vivant à sang chaud.

8. Trousse pour la préparation d'une composition radiopharmaceutique, comprenant (i) un petit peptide ayant une affinité sélective pour les récepteurs de neurokinine 1 et muni éventuellement d'un groupe de liaison tel que défini dans la revendication 1, cette substance étant, si désiré, additionnée d'un support inerte pharmaceutiquement acceptable et/ou d'agents de formulation et/ou d'adjuvants, (ii) une solution d'un composé d'un radionucléide choisi dans le groupe constitué par Pb-203, Ga-67, Ga-68, As-72, In-111, In-113m, Ru-97, Tc-99m, Re-186, Re-188, Cu-62, Cu-64, Fe-52, Mn-52m, Cr-51, I-123, 1-131, Br-75, Br-76, As-77, Y-90, Cu-67, Er-169, Sn-121, Te-127, Pr-142, Pr-143, Au-198, Pd-109 et Dy-165, et (iii) un mode d'emploi avec une prescription pour la réaction des ingrédients présents dans la trousse.

9. Trousse pour la préparation d'une composition radiopharmaceutique comprenant (i) un petit peptide ayant une affinité sélective pour les récepteurs de neurokinine 1 et muni éventuellement d'un groupe de liaison tel que défini dans la revendication 1, cette substance étant, si désiré, additionnée d'un support inerte pharmaceutiquement acceptable et/ou d'agents de formulation et/ou d'adjuvants, (ii) un agent réducteur et, si désiré, un agent chélatant, lesdits ingrédients (i) et (ii) étant éventuellement mélangés, et (iii) un mode d'emploi avec une prescription pour la réaction des ingrédients de la trousse avec du Tc-99m sous forme d'une solution de pertechnétate ou avec du Re-186 ou du Re-188 sous forme d'une solution de perrhénate.

10. Trousse selon la revendication 8 ou la revendication 9, comprenant un conjugué de peptide, obtenu par la modification d'un petit peptide, tel que défini dans la revendication 1, par réaction avec un lieur ayant un groupe fonctionnel pour la liaison ou la chélatation d'un radionucléide tel que défini dans la revendication 1.

11. Trousse selon la revendication 10, comprenant un conjugué de peptide obtenu par modification dudit petit peptide par réaction avec un lieur, ledit lieur étant dérivé de l'acide éthylènediaminetétracétique (EDTA), de l'acide diéthylènetétraminepentacétique (DPTA), de l'acide éthylèneglycol-O,O'-bis(2-aminoéthyl)-N,N,N',N'-tétracétique (EGTA), de l'acide N,N'-bis(hydroxybenzyl)-éthylènediamine-N,N'-diacétique (HBED), de l'acide triéthylènetétraminehexacétique (TTHA), d'EDTA substitué ou de DTPA substitué, de l'acide 1,4,7,10-tétra-azacyclododécane-N,N',N'',N'''-tétracétique (DOTA) ou de l'acide 1,4,8,11-tétra-azacyclotétradécane-N,N',N",N"'-tétracétique (TETA), ou d'un composé de formule générale 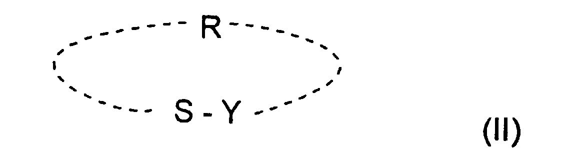 dans laquelle R est un radical hydrocarbyle linéaire ou ramifié, éventuellement substitué, qui peut être interrompu par un ou plusieurs hétéroatomes choisis parmi N, O et S et/ou par un ou plusieurs groupes NH, et Y est un groupe capable de réagir avec un groupe amino du peptide et choisi de préférence dans le groupe constitué par les groupes carbonyle, carbimidoyle, N-(alkyl en C₁-C₆)carbimidoyle, N-hydroxycarbimidoyle et N-(alcoxy en C₁-C₆)carbimidoyle.
